(19) Europäisches Patentamt
European Patent Office
Office européen des brevets

(11) Publication number: 0 226 664
A1

(12) **EUROPEAN PATENT APPLICATION**

(21) Application number: 85201961.1

(22) Date of filing: 26.11.85

(51) Int. Cl.4: G03C 7/32 , G03C 5/54 , //C07D231/52,C07D211/58

(43) Date of publication of application: 01.07.87 Bulletin 87/27

(84) Designated Contracting States: AT BE CH DE FR GB IT LI LU NL SE

(71) Applicant: AGFA-GEVAERT naamloze vennootschap
Septestraat 27
B-2510 Mortsel(BE)

(72) Inventor: Monballiu, Marcel Jacob
Neuris 9
B-2510 Mortsel(BE)
Inventor: Van Meerbeeck, Paul Louis
Langestraat 103
B-3141 Hulsthout(BE)

(54) **Photographic element comprising ballasted compounds.**

(57) The present invention relates to a photographic element comprising in a hydrophilic colloid layer a ballasted compound comprising a ballasting moiety that renders such compound fast to diffusion in said hydrophilic colloid layer and a photographically useful moiety linked to this ballasting moiety, the ballasting moiety corresponding to the general formula:

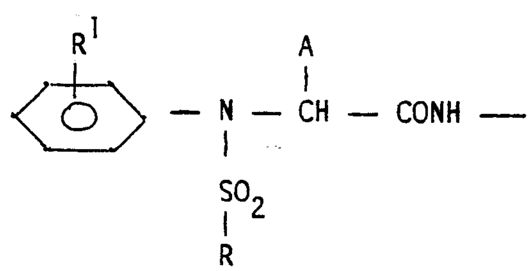

(I)

wherein A is hydrogen, $C_1$-$C_{20}$ alkyl, or aryl; R is $C_1$-$C_{20}$ alkyl; $R^1$ is hydrogen, hydroxy, sulpho, carboxy, cyano, a halogen atom, trifluoromethyl, hydroxyalkyl, sulphoalkyl, carboxyalkyl, alkyl, alkoxy, alkylthio, acyl, $R^2CONH$-, $R^2NHCO$-, $R^2NHSO_2$ , or $R^2SO_2N(R^3)$-, each of $R^2$ and $R^3$ being hydrogen, $C_1$-$C_5$ alkyl, or aryl and when both present having a same or a different significance.
The present invention also relates to compounds comprising such ballasting moiety.

EP 0 226 664 A1

# PHOTOGRAPHIC ELEMENT COMPRISING BALLASTED COMPOUNDS

The present invention relates to a photographic element comprising at least one compound comprising a ballasting moiety that renders such compound substantially fast to diffusion in a hydrophilic colloid medium and at least one photographically useful moiety that is linked chemically to this ballasting moiety. The present invention also relates to compounds comprising such ballasting moiety.

In photography in general and in colour photography and diffusion transfer processes in particular it is often necessary that at least some of the photographically useful ingredients or compounds of a photographic element remain steadily fixed in the hydrophilic layer or layers, in which they have been incorporated initially.

In order to obtain excellent colour images, colour separation has to be perfect and therefore it is essential indeed that colour couplers and mask-forming compounds, when incorporated into photographic light-sensitive emulsions, remain perfectly immobile in the hydrophilic colloid layer, in which they have been incorporated originally, and that they do not start wandering or diffusing through the photographic element.

The problem of inter-layer diffusion of photographically useful compounds is well-known in the art and has been described extensively in a great many of patents and other publications. From the latter publications one can learn that diffusion of photographically useful compounds through hydrophilic colloid layers can be avoided advantageously by providing said photographically useful compounds with at least one ballasting moiety. In general such ballasting moieties consist of or comprise long branched or unbranched aliphatic hydrocarbon chains of e.g. from 5 to 20 or more carbon atoms. Their incorporation into photographically useful compounds involves no particular synthetical problems. Examples of ballasting moieties known to be relatively useful in photography can be found in e.g. DE-A 2,418,959 and GB-A 1,336,171.

The ballasting moieties impart a hydrophobic character to the molecules of the photographically useful compounds. The ballasted compounds are incorporated into hydrophilic colloid compositions either in dispersed form or in dissolved form. In the latter case the ballasted compounds should be provided with solubilizing groups such as e.g. carboxy, sulpho, and hydroxy.

More frequently, however, the ballasted compounds are incorporated into the hydrophilic colloid media according to well-known dispersion techniques. For this purpose the compounds can be dissolved in a low-boiling or high-boiling water-immiscible solvent or mixture thereof, the resulting solution being dispersed into water or into an aqueous solution of a hydrophilic colloid with the aid of one or more dispersing agents. After removal of the low-boiling solvent the dispersion is admixed with the coating composition of the hydrophilic colloid layer of a photographic element.

Photographically useful compounds, which in the manufacture of photographic silver halide elements have to be incorporated into one or more hydrophilic colloid layers of such elements as ballasted compounds, include i.a. couplers, competing couplers, mask-forming compounds, UV-absorbers, stabilizers, anti-oxidants, anti-fading agents, and dyes.

By coupler is meant any compound, which in silver halide photography couples with an oxidized aromatic primary amino colour developing agent to form a dye image.

Competing couplers are compounds, which are used in conjunction with couplers in silver halide colour photography and which couple with the oxidized aromatic primary amino colour developing agent to form colourless coupling products as described in e.g. GB-A 861,138.

By mask-forming compounds are meant compounds, which oxidatively couple with couplers in an oxidizing bleaching bath to form coloured mask images as described in e.g. GB-A 880,862 and 975,932.

As is well-known from all types of diffusion transfer processes (DTR-processes) certain compounds such as e.g. dyes, couplers, redox-dye releasing compounds, electron-donor compounds, scavenger compounds for oxidized developing agents, etc. have to be retained in their original hydrophilic colloid layer, at least for a controllable period of time.

Several dye-diffusion transfer systems are described, e.g., in GB-A 1,243,048 and 1,405,662; US-A 3,227,550 -3,628,952 -4,030,920 -4,139,379 -4,139,389; published US Ser. B 351,673; DE-A 2,645,656; EP-A 0004399 -0004399 -0038092; and GB-A 80 31,433.

Compounds of the type referred to hereinbefore and provided with the conventional well-known ballasting moieties present some drawbacks in that they often crystallize from their dispersions and in that the dispersions are too coarse.

In EP-A 0,073,636 couplers are described, which contain attached to a position other than the coupling position a ballasting moiety terminated with a hydroxyphenylsulphonyl or hydroxyphenylsulphinyl group. Although an improvement in the colour formation is obtained with these couplers, the extent of the improvement is still insufficient. Moreover, these couplers have a low solubility in organic solvents and consequently are difficult to disperse.

In US-A 4,503,141 couplers are described, which carry an analogous ballasting moiety but wherein the phenyl group of the hydroxyphenylsulphonyl or hydroxyphenylsulphinyl group is replaced by an aromatic heterocyclic ring. However, these couplers can be synthesized only wih great difficulty and as a consequence, they are hardly suitable from an economical standpoint.

It is an object of the present invention to provide a photographic element, which in a hydrophilic colloid layer comprises a ballasted compound that in dispersed state in the hydrophilic colloid does not present the above drawbacks.

The present invention provides a photographic element, which in at least one hydrophilic colloid layer comprises at least one ballasted compound comprising a ballasting moiety that renders such compound substantially fast to diffusion in said hydrophilic colloid layer and at least one photographically useful moiety that is linked chemically to this ballasting moiety characterized in that the ballasting moiety of the ballasted compound corresponds to the following general formula I:

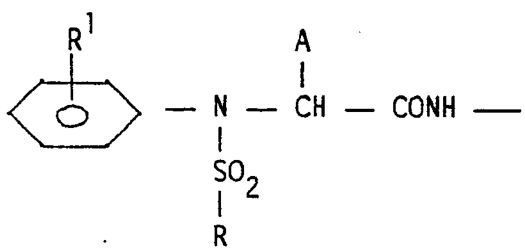 (I)

wherein:

A represents hydrogen, a straight chain or branched chain $C_1$-$C_{20}$ alkyl group e.g. ethyl, isopropyl, n-butyl, and n-dodecyl, or an aryl group;

R is a $C_1$-$C_{20}$ alkyl group e.g. methyl, n-dodecyl, and n-hexadecyl;

$R^1$ represents hydrogen, hydroxy, sulpho, carboxy, cyano, a halogen atom e.g. chloro, trifluoromethyl, a hydroxyalkyl group, a sulphoalkyl group, a carboxyalkyl group, an alkyl group e.g. methyl, an alkoxy group e.g. o-hexadecyloxy, an alkylthio group, an acyl group e.g. palmitoyl, $R^2CONH$-, $R^2NHCO$-, $R^2NHSO_2$ , or $R^2SO_2N(R^3)$-, each of $R^2$ and $R^3$ being hydrogen, a

$C_1$-$C_5$ alkyl group, or an aryl group and when both present having a same or a different significance.

The ballasted compounds used in accordance with the present invention comprise at least one photographically useful group. Such photographically useful group can be any group used in a photographic process, i.a. a colour coupler group, a competing coupler group, a mask-former group, an UV-absorber group, a stabilizer group, an anti-oxidant group, an anti-fading group, a dye group, and in the case of a DTR-process i.a. a redox-controlled dye-releasing group, an electron-donor group, and a scavenger group for oxidized developing agents. An anti-fading group can be useful in preserving the stability of dyes obtained by development or by diffusion transfer.

It was found that compounds bearing a ballasting moiety corresponding to the above general formula and at least one photographically useful moiety that is linked chemically to this ballasting moiety offer some particularly interesting advantages over similar compounds comprising (a) same photographically useful group(s) but having a ballasting moiety of a different type. The ballasted compounds used in accordance with the present invention and comprising at least one photographically useful moiety, whichever the intrinsic structure thereof may be, can be dispersed easily in water or in aqueous hydrophilic colloid media. This makes it possible to prepare with great success highly concentrated aqueous dispersions of such compounds according to the methods referred to hereinbefore and described e.g. in US-A 3,658,546; DE-AS 1,127,714; US-A 2,322,027, and GB-A 1,297,947.

Furthermore, it has been established surprisingly that couplers comprising a ballasting moiety corresponding to the above general formula I, while being readily dispersible in a hydrophilic colloid medium e.g. in a hydrophilic colloid layer of a photographic element, yield, upon image-wise exposure and colour development of such photographic element, a dye image therein that shows a substantially increased gradation and maximum density when compared with couplers comprising a known ballasting moiety.

The ballasting moiety of compounds used in accordance with the present invention has a favourable influence upon the micro-structure of a dispersion of such ballasted compounds.

It was also found that thanks to the introduction of a ballasting moiety corresponding to the above general formula into antifading agents used for preserving the stability of dyes obtained by development or by diffusion transfer, the antifading influence of such ballasted antifading agents upon such dyes was enhanced considerably.

Examples of ballasted compounds that can be used in accordance with the present invention are listed in the following tables.

TABLE 1

Cyan-forming phenol colour couplers

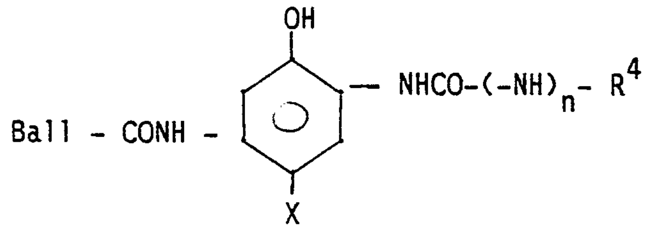

| Coupler | Ball | $R^4$ | n | X | Melting at °C |
|---|---|---|---|---|---|
| C1 | 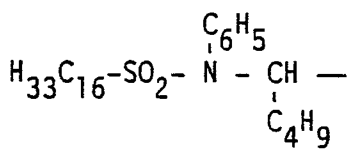 | $C_6H_5$ | 0 | Cl | 140. |
| C2 | idem | $C_6H_4Cl$-4 | 0 | Cl | 149 |
| C3 | idem | $C_6H_4Cl$-2 | 0 | Cl | 100 |
| C4 | idem | 2-furyl | 0 | Cl | 147 |
| C5 | idem | $C_6H_4F$-4 | 1 | H | 190 |
| C6 | 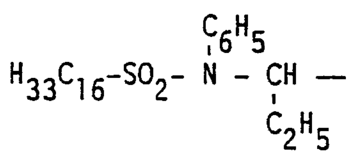 | $C_6H_5$ | 0 | Cl | 121 |
| C7 | idem | $C_6H_4Cl$-2 | 0 | Cl | 100 |
| C8 | idem | $(CF_2)_2-CF_3$ | 0 | H | 80 |
| C9 | 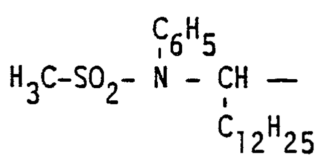 | $C_6H_5$ | 0 | Cl | 155 |

| | | | | | |
|---|---|---|---|---|---|
| C10 | idem | $C_6H_4Cl$-4 | 0 | Cl | 162 |
| C11 | idem | $C_6H_4Cl$-2 | 0 | Cl | 129 |
| C12 | idem | $C_6H_4-C_4H_9$t.-4 | 0 | Cl | 144 |
| C13 | idem | 2-furyl | 0 | Cl | 183 |
| C14 | idem | $C_6H_4F$-4 | 1 | H | 177 |
| C15 | $C_6H_4-O-C_{16}H_{33}-2$<br>$H_3C-SO_2-$ N - CH —<br>$C_2H_5$ | $C_6H_5$ | 0 | Cl | 103 |
| C16 | idem | $C_6H_4Cl$-2 | 0 | Cl | 126 |
| C17 | idem | $C_6H_4F$-4 | 1 | H | 159 |
| C18 | $C_6H_4$- $COC_{15}H_{31}$-4<br>$H_{33}C_{16}-SO_2-$ N - CH —<br>$C_2H_5$ | $C_6H_4Cl$-2 | 0 | Cl | 157 |
| C19 | 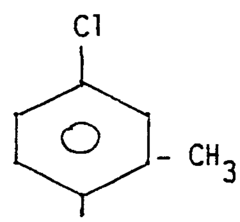<br>$H_{33}C_{16}-SO_2-$ N - CH —<br>$C_2H_5$ | $C_6H_4Cl$-2 | 0 | Cl | 65-66 |
| C20 | idem | $C_6H_5$ | 0 | Cl | 132 |

TABLE 2

Cyan-forming phenol colour couplers

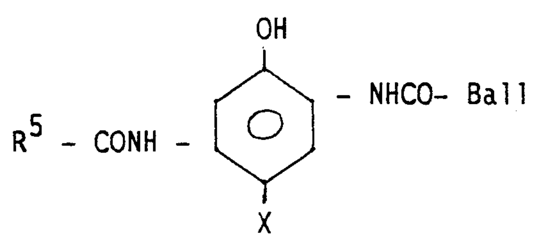

| Coupler | Ball | $R^5$ | X | Melting at °C |
|---|---|---|---|---|
| C21 | $C_6H_5$<br>$H_3C-SO_2-$ N - CH —<br>$C_{12}H_{25}$ | $C_4H_9$-t. | Cl | 114 |

| | | | | |
|---|---|---|---|---|
| C22 | idem | 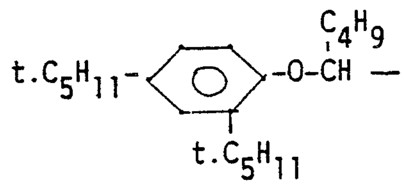 | Cl | 138 |
| C23 | idem | idem | H | oil |

TABLE 3

Cyan-forming phenol colour coupler

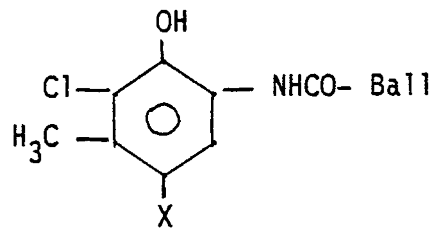

| Coupler | Ball | X | Melting at °C |
|---|---|---|---|
| C24 | 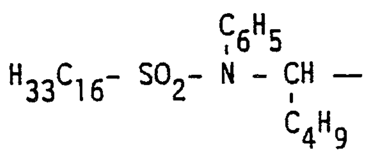 | Cl | 92 |

TABLE 4

Cyan-forming phenol colour coupler

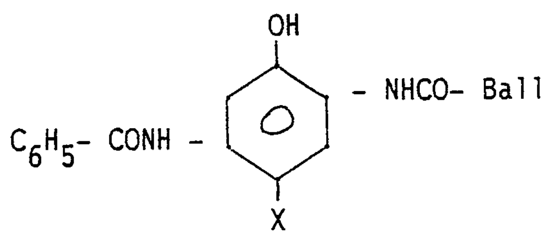

| Coupler | Ball | X | Melting at °C |
|---|---|---|---|
| C25 | 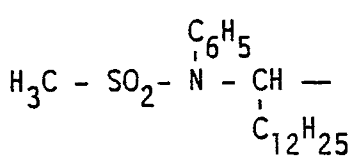 | H | 206 |

TABLE 5

Cyan-forming naphthol colour coupler

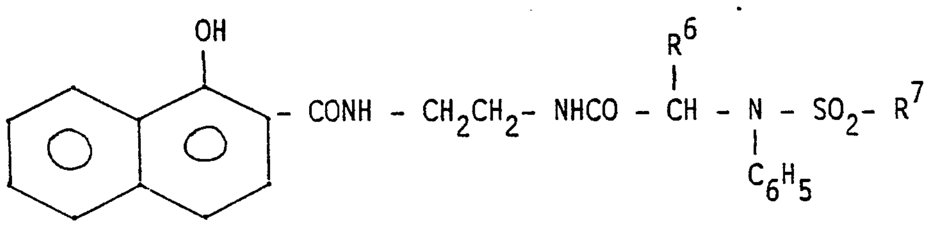

| Compound | $R^6$ | $R^7$ | Melting at °C |
|---|---|---|---|
| C26 | $C_4H_9$ | $C_{16}H_{33}$ | oil |

TABLE 6

Magenta-forming 2-pyrazolin-5-one colour couplers

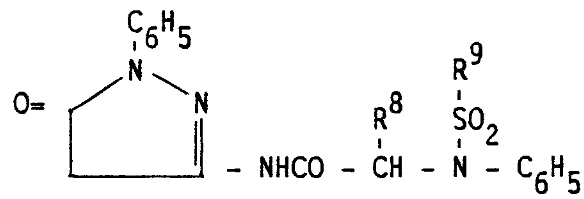

| Coupler | $R^8$ | $R^9$ | Melting at °C |
|---|---|---|---|
| M1 | $C_4H_9$ | $C_{16}H_{33}$ | 83 |
| M2 | $C_2H_5$ | idem | 76 |
| M3 | $C_{12}H_{25}$ | $CH_3$ | 110 |

TABLE 7

Magenta-forming 2-pyrazolin-5-one colour couplers

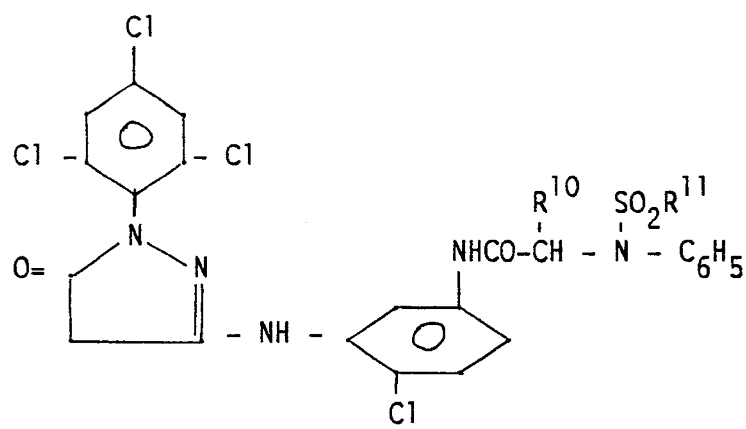

| Coupler | $R^{10}$ | $R^{11}$ | Melting at °C |
|---|---|---|---|
| M4 | $C_{12}H_{25}$ | $CH_3$ | 92 |
| M5 | $C_4H_9$ | $C_{16}H_{33}$ | 102 |
| M6 | $C_2H_5$ | idem | 130 |

TABLE 8

Magenta-forming 2-pyrazolin-5-one colour coupler

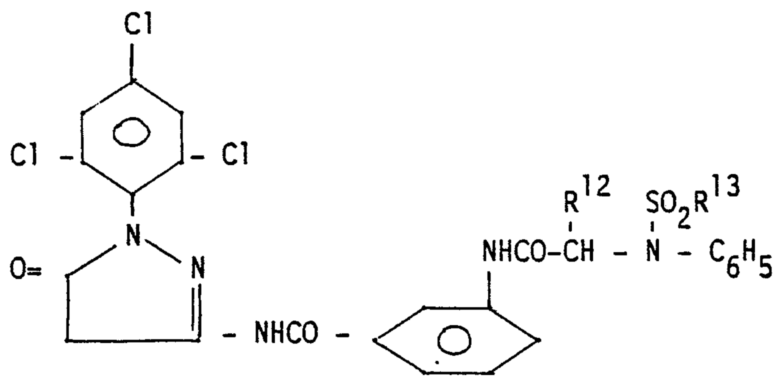

| Coupler | $R^{12}$ | $R^{13}$ | Melting at °C |
|---|---|---|---|
| M7 | $C_{12}H_{25}$ | $CH_3$ | 188 |

TABLE 9

Magenta-forming 2-pyrazolin-5-one colour couplers

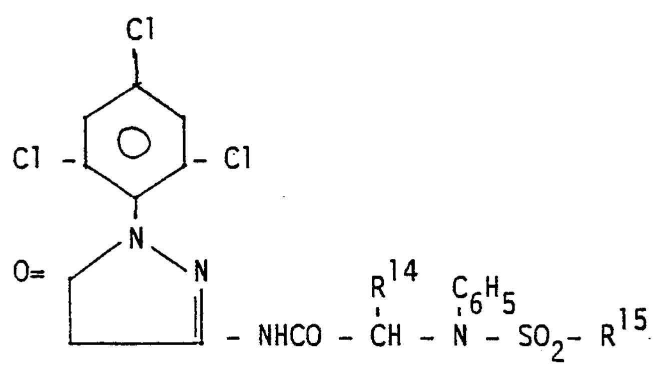

| Coupler | $R^{14}$ | $R^{15}$ | Melting at °C |
|---|---|---|---|
| M8 | $C_2H_5$ | $C_{16}H_{33}$ | lower than 50 |
| M9 | $C_4H_9$ | $C_{16}H_{33}$ | lower than 50 |
| M10 | $C_{12}H_{25}$ | $CH_3$ | 127 |

TABLE 10

Yellow-forming acetoacetanilide colour coupler

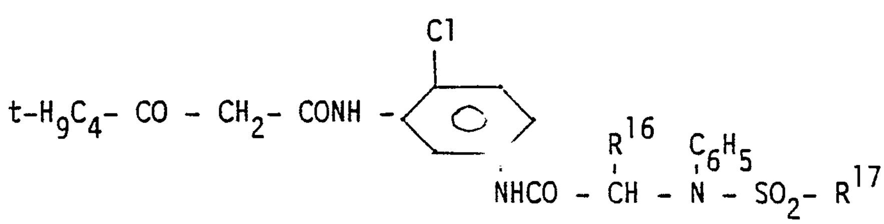

| Coupler | $R^{16}$ | $R^{17}$ | Melting at °C |
|---|---|---|---|
| Y1 | $C_4H_9$ | $C_{16}H_{33}$ | 97 |

TABLE 11

Anti-fading compound

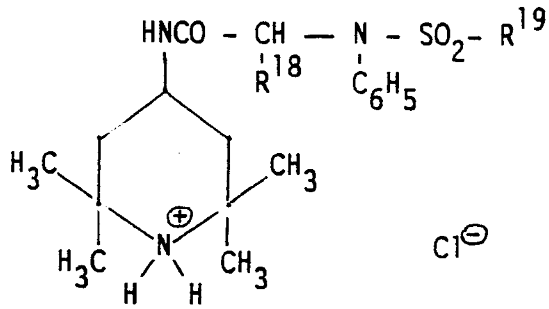

| Compound | $R^{18}$ | $R^{19}$ | Melting at °C |
|---|---|---|---|
| A1 | $C_2H_5$ | $C_{16}H_{33}$ | 169-171 |

TABLE 12

Stabilizer

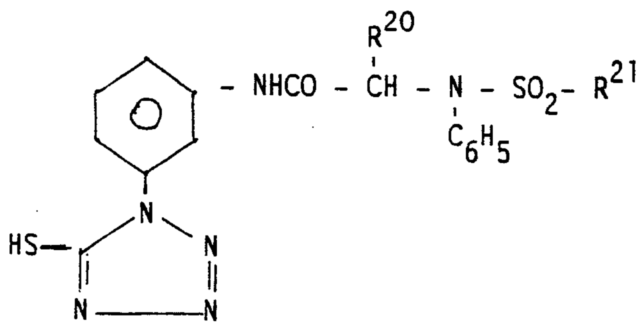

| Compound | $R^{20}$ | $R^{21}$ | Melting at °C |
|---|---|---|---|
| S1 | $C_2H_5$ | $C_{16}H_{33}$ | 87-91 |

TABLE 13

Competing coupler

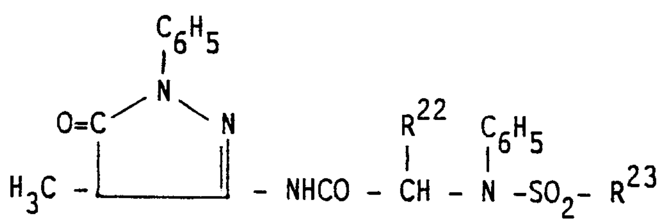

| Compound | $R^{22}$ | $R^{23}$ | Melting at °C |
|---|---|---|---|
| P1 | $C_2H_5$ | $C_{16}H_{33}$ | oil |

Starting compounds for forming the ballasting moieties during the synthesis of the ballasted compounds for use in accordance with the present invention can be prepared as illustrated by the following reaction sequence, wherein the symbols A, R, and R' have significances as defined in the above general formula I and wherein Y stands for an alkyl group:

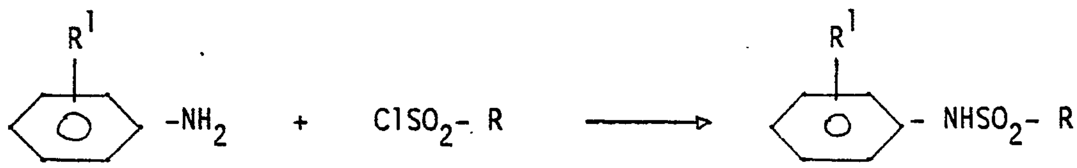

(II)

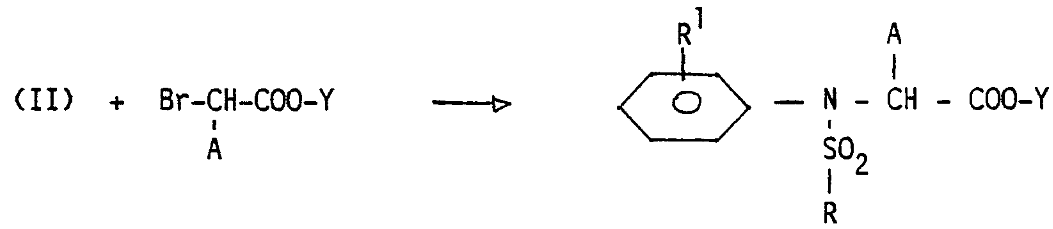

(III)

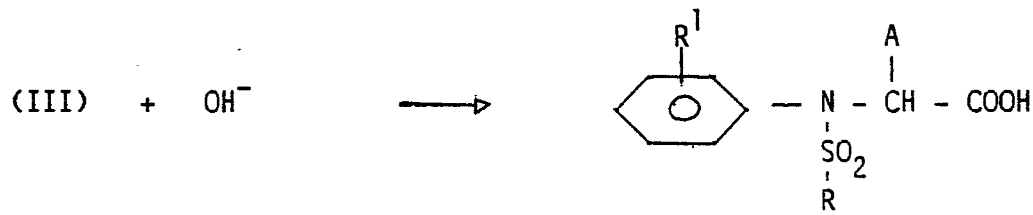

(IV)

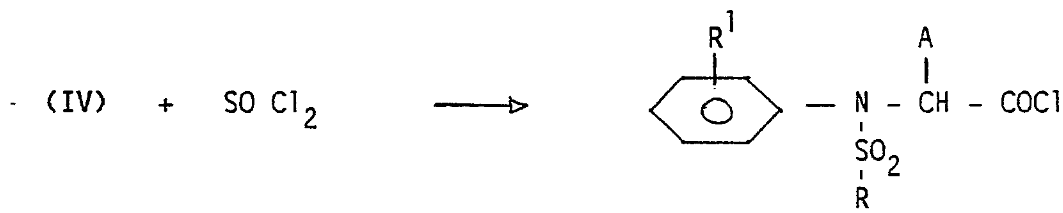

(IV)

Other starting compounds for forming the ballasting moieties according to the present invention can be prepared analogously or by techniques known in the art starting with the appropriate chemicals.

The following preparation illustrates the synthesis of a starting compound for forming the ballasting moiety in ballasted compounds for use in accordance with the present invention.

Preparation of 2-(N-phenyl-N-n-hexadecylsulphonyl-amino)-hexanoyl chloride.

a) n-Hexadecyl-sulphonyl anilide

An amount of 683 g (2 mol) of n-hexadecyl sulphochloride was added to a solution of 186 g (2 mol) of aniline in 176 ml (2.2 mol) of pyridine and 4 l of acetonitrile. The solution was refluxed for 1 h and the resulting precipitate was filtered with suction. The product was stirred in 1.5 l of ethanol and 50 ml of acetic acid and then filtered again with suction. Finally, it was recrystallized from 4 l of cyclohexane.
Yield: 664 g (87%) of n-hexadecyl-sulphonyl anilide.

b) 2-(N-phenyl-N-n-hexadecylsulphonyl-amino)-hexanoic acid

An amount of 250 g (1.2 mol) of 2-bromo-hexanoic acid methyl ester was added with stirring to a suspension of 381 g (1 mol) of n-hexadecyl-sulphonyl anilide and 166 g of potassium carbonate in 1500 ml of dimethylformamide. The reaction mixture was heated for 2 h at 90-95°C. The inorganic salts were filtered off. The filtrate was diluted with 2 kg of solid ice and acidified with 5N hydrochloric acid to pH 1. The oil obtained became solid. The precipitate was filtered and dissolved in 1500 ml of methanol. A solution of 120

g of sodium hydroxide in 250 ml of water was added thereto. The resulting solution was heated for 1 h at 60°C, acidified to pH 1, and extracted with 2 l of dichloromethane. The extract was dried over magnesium sulphate. The organic sovent was removed by evaporation. A yellowish oil was obtained, which upon standing became solid.
Yield: 470 g (94%) of 2-(N-phenyl-N-n-hexadecylsulphonyl-amino)-hexanoic acid.

c) 2-(N-phenyl-N-n-hexadecylsulphonyl-amino)-hexanoyl chloride

An amount of 470 g (0.95 mol) of 2-(N-phenyl-N-n-hexadecylsulphonyl-amino)-hexanoic acid was dissolved in 300 ml of thionyl chloride. The solution was heated for 1 h at 50°C. The excess thionyl chloride was removed by evaporation. A yellowish oil was obtained.
Yield: 490 g (100%) of 2-(N-phenyl-N-n-hexadecylsulphonyl-amino)-hexanoyl chloride.

The following preparation illustrates the synthesis of a ballasted magenta-forming 2-pyrazolin-5-one colour coupler as well as of a ballasted antifading agent, both for use in accordance with the present invention.

Preparation of magenta-forming coupler M5

An amount of 60.6 g (0.15 mol) of 1-(2,4,6-trichloro)-phenyl-3-(2-chloro-5-amino)-anilino-2-pyrazolin-5-one and 83.2 g (0.16 mol) of 2-(N-phenyl-N-n-hexadecylsulphonyl-amino)-hexanoyl chloride was added to 500 ml of acetonitrile. The reaction mixture was heated for 4 h at 80°C and poured out in 1N hydrochloric acid. The solution was extracted with dichloromethane and dried over magnesium sulphate. The residual oil was purified chromatographically.
Yield: 83 g (63%) of magenta-forming coupler M5. Melting point: 102°C.

Preparation of antifading agent A1

An amount of 19.42 g (0.4 mol) of 2-(N-n-hexadecylsulphonyl-N-phenyl)-amino-butyryl chloride in 25 ml of dichloromethane was added dropwise in 15 min to a solution of 6.84 ml (0.04 mol) of 2,2,6,6-tetramethyl-4-aminopiperidine in 25 ml of dichloromethane with cooling so as to keep the reaction temperature between 20 and 25°C. A white crystalline precipitate formed. After 15 min of stirring the reaction mixture was diluted with 150 ml of 1,1,2,trifluoro-2,2,1-trichloro-ethane. The precipitate was filtered with suction. The filtrate was recrystallized from acetonitrile.
Yield: 14.5 g (56%) of antifading agent A1. Melting point: 169-171°C.

Other ballasted compounds for use in accordance with the present invention, the ballasting moiety of which corresponds to the above general formula I can be prepared analogously or by techniques known in the art of introducing ballasting moieties into photographically useful compounds.

The compounds comprising a ballasting moiety corresponding to the above general formula I are believed to be new compounds and the present invention also includes such compounds per se.

The compounds comprising a ballasting moiety corresponding to the above general formula I can be incorporated into any type of photographic element comprising a light-sensitive silver halide emulsion layer, e.g. a spectrally sensitized silver halide emulsion layer, a non-spectrally sensitized silver halide emulsion layer, a silver halide emulsion layer of use in diffusion transfer reversal processes, an X-ray silver halide emulsion layer, a lith silver halide emulsion layer, or a silver halide emulsion layer sensitive to infra-red radiation. Various silver salts can be used as the light-sensitive silver salt e.g. silver bromide, silver iodide, silver chloride, and mixed halides e.g. silver chlorobromide, silver chlorobromoiodide, and silver bromoiodide.

The compounds comprising a ballasting moiety corresponding to the above general formula I and a photographically useful moiety having a function in the formation of an image according to a diffusion transfer reversal process can also be incorporated into a non-light-sensitive element comprising an image-receiving layer.

The expression "photographic element" as used herein consequently includes besides light-sensitive elements also non-light-sensitive elements such as image-receiving elements as used in diffusion transfer reversal processes.

Compounds for use in accordance with the present invention, which can be incorporated into a non-light-sensitive element comprising an image-receiving layer, are e.g. compounds comprising as photographically useful moiety an anti-fading group that preserves the stability of dyes obtained by diffusion transfer.

The ballasted compounds for use in accordance with the present invention are preferably incorporated into a hydrophilic colloid medium from a solution in at least one high-boiling sparingly water-miscible solvent e.g. di-n-butyl phthalate and tricresyl phosphate or in a low-boiling sparingly water-miscible solvent e.g. ethyl acetate, methylene chloride, and chloroform, or in mixtures thereof. Very fine dispersions of ballasted compounds according to the invention in hydrophilic colloid media can be obtained by means of these solvents. For this purpose these solutions are dispersed in extremely fine droplets, preferably in the presence of a wetting or dispersing agent, into the hydrophilic colloid medium, the low-boiling sparingly water-miscible solvent being removed afterwards by evaporation.

The ballasted compounds for use in accordance with the present invention can, of course, also be incorporated in other ways into hydrophilic colloid media. For instance, when the compound can be liquefied by slight heating (in case it has a low melting point) or when it is liquid at room temperature, the liquid can be dispersed as such into the hydrophilic colloid media.

The hydrophilic colloid media, into which the ballasted compounds for use in accordance with the present invention can be dispersed, need not necessarily be coating compositions for hydrophilic colloid layers. Especially in the case of photographic emulsion components that are to be incorporated into light-sensitive silver halide emulsions, it may be advantageous to first disperse these components into non-light-sensitive hydrophilic colloid compositions or into water, and subsequently to admix the resulting dispersions in their turn with the colloid coating composition such as a silver halide emulsion, from which the hydrophilic colloid layer is to be coated. The hydrophilic colloid media, into which the ballasted compounds according to the invention can be incorporated, usually comprise gelatin as hydrophilic colloid. However, other water-soluble colloid materials or mixtures thereof can be used too e.g. colloidal albumin, zein, casein, a cellulose derivative such as carboxymethylcellulose, or a synthetic hydrophilic colloid such as polyvinyl alcohol, and poly-N-vinylpyrrolidone.

For more details about suitable dispersing techniques that can be employed for incorporating the ballasted compounds according to the invention into a hydrophilic colloid layer of a photographic element, reference can be made to e.g. US-A 2,269,158 -2,284,887 -2,304,939 -2,304,940, and 2,322,027; GB-A 791,219; FR-A 1,555,663; DE-A 1,127,714, and to BE-A 747,589.

The photographically useful compounds ballasted according to the present invention are particularly suited for being dispersed in water, even in relatively high concentrations, which obviously can be very important for manufacturing purposes. The use of the ballasting moieties corresponding to the above general formula I makes it even possible to prepare highly concentrated aqueous dispersions that are free of gelatin in a systematic and easy way and substantially irrespective of the structure of the remainder of the molecule according to the method set forth in US-A 3,658,546.

The silver halide emulsions of light-sensitive photographic elements according to the present inventioncan be sensitized chemically as well as spectrally. They can be sensitized chemically by effecting the ripening in the presence of small amounts of sulphur-containing compounds such as allyl thiocyanate, allylthiourea, and sodium thiosulphate. The emulsions may also be sensitized by means of reductors e.g. tin compounds as described in FR-A 1,146,955 and in BE-A 568,687, imino-amino methane sulphinic acid compounds as described in GB-A 789,823 and small amounts of noble metal compounds such as gold, platinum, palladium, iridium, ruthenium, and rhodium compounds. They can be sensitized spectrally by means of cyanine and merocyanine dyes.

The light-sensitive silver halide emulsions can also comprise compounds that sensitize the emulsions by development acceleration, e.g. compounds of the polyoxyalkylene type such as alkylene oxide condensation products as described i.a. in US-A 2,531,832 -2,533,990 -3,210,191, and 3,158,484, in GB-A 920,637 and 991,608, and in BE-A 648,710, and onium derivatives of amino-N-oxides as described in GB-A 1,121,696.

Further, the emulsions may comprise stabilizers, e.g. heterocyclic nitrogen-containing thioxo-compounds such as benzothiazoline-2-thione and 1-phenyl-2-tetrazoline-5-thione and compounds of the hydroxytriazolopyrimidine type. They can also be stabilized with mercury compounds such as the mercury compounds described in BE-A 524,121 -677,337, and 707,386 and in US-A 3,179,520.

The light-sensitive emulsions can also comprise all other kinds of ingredients such as plasticizers, hardening agents, and wetting agents.

The emulsions can be coated on a wide variety of photographic emulsion supports. Typical supports include cellulose ester film, polyvinylacetal film, polystyrene film, polyethylene terephthalate film and related films of resinous materials, as well as paper e.g. polyethylene-coated paper, and glass.

The following examples illustrate the invention.

EXAMPLE 1

An amount of 1 g of ballasted colour coupler as defined in Table 14 hereinafter was dissolved in 5 ml of ethyl acetate at 75°C. By means of a homogenizer the resulting solution was dispersed at 40°C in 9 ml of an aqueous solution of gelatin (2.5 g) and 1 ml of a 10 % aqueous solution of the wetting agent n-dodecyl-benzene sulphonic acid sodium salt.

The ethyl acetate was removed substantially by evaporation under reduced pressure (27 -53 kPa) at 55°C. Distilled water was added to make a total volume of 10 ml. In this way an aqueous dispersion of 10% by weight of colour coupler suited for admixture with a conventional gelatin silver halide emulsion ready for coating was obtained.

A comparison between dispersion results obtained with the ballasted colour couplers C6, M8, and M9 according to the invention and dispersion results obtained with analogous colour couplers carrying a known ballasting moiety (CR1, MR1, and MR2) is made in the following Table 14.

TABLE 14

| Ballasted compound | Dispersible | Stability after 24 h at 40°C | Stability after 1 week at 40°C |
|---|---|---|---|
| CR1 (comparison) | yes | partially crystallizing | crystallizing |
| Compound C6 | yes | good | good |
| MR1 (comparison) | yes | partially crystallizing | partially crystallizing |
| Compound M8 | yes | good | good |
| MR2 (comparison) | yes | partially crystallizing | crystallizing |
| Compound M9 | yes | good | good |

The comparison couplers CR1, MR1, and MR2 correspond to the following structural formulae respectively:

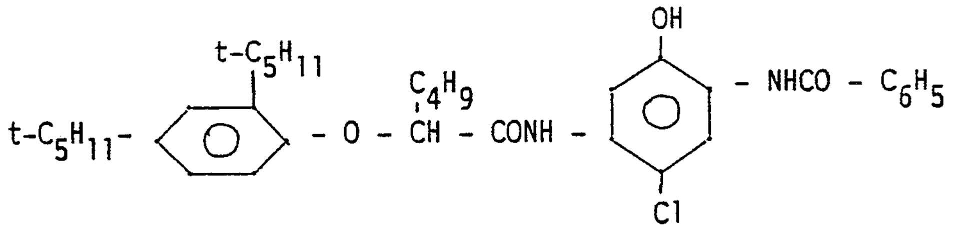

CR1

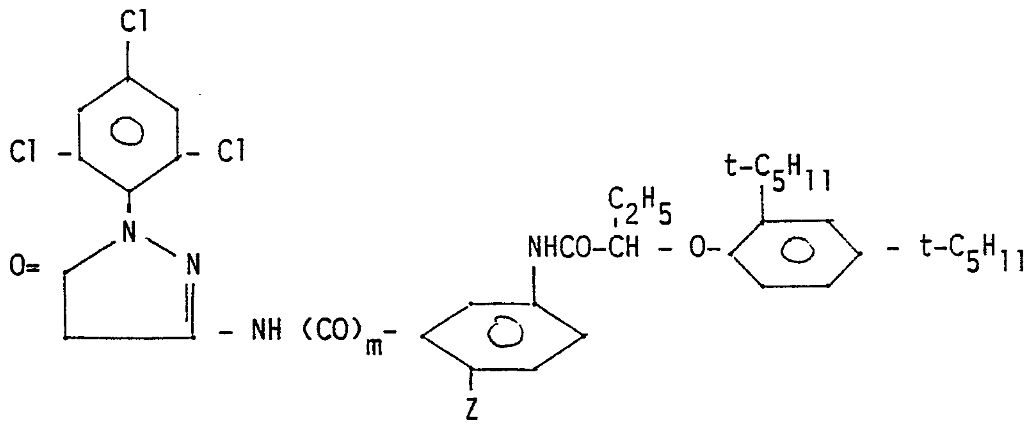

wherein Z is H and m is 1 in the case of MR1, and
Z is Cl and m is 0 in the case of MR2.

The results listed in Table 14 show that the ballasted compounds according to the invention can be dispersed more successfully than structurally analogous compounds comprising a conventional ballasting moiety.

EXAMPLE 2

Ballasted colour couplers as listed in Table 15 hereinafter were dispersed in a hydrophilic colloid medium according to the following technique.

83 ml of 5% aqueous gelatin was mixed with 5 to 10 % of an emulgator based on the weight of gelatin. The mixture was dispersed at 40°C. Within 30 s a solution of 6 mmol of the colour couplers and an equal weight of dibutyl phthalate in 18-36 ml of ethyl acetate were added with stirring. The acetic ester was then removed from the mixture by bringing the latter into a rotating thin-layer evaporator at 60°C and about 5 kPa.

An amount of 110 g of a silver bromo-iodide emulsion (2.3 % iodide) having a gelatin content of 85.8 g per kg and a silver halide content corresponding to 50 g of silver nitrate per kg was mixed in molten state with:
154.4 g of 7.5 % aqueous gelatin, whereupon water was added in an amount of
100 ml for a cyan-forming coupler or of
200 ml for a magenta-forming coupler.

The mixture was left standing at 40°C for 1 h. Next, 6 mmol of the colour coupler dispersion was added with stirring together with the usual additives such as hardeners, wetting agents and stabilizers. Finally, water was added to make a total weight of 575 g (for a cyan-forming coupler) or 720 g (for a magenta-forming coupler).

The solution was coated on a cellulose triacetate support at a ratio of 125 g per m2.

After exposure to light of the appropriate wavelength through a grey wedge, the photographic elements obtained were developed at 24°C for 10 min in a colour developing bath having the following composition:

| | |
|---|---|
| sodium hexametaphosphate | 2 g |
| anhydrous sodium sulphite | 4 g |
| anhydrous sodium carbonate | 17 g |
| potassium bromide | 2 g |
| 2-amino-5-diethylaminotoluene hydrochloride (CD 2) | 3 g |
| water to make | 1 l |

Next, the developed elements were treated at 24°C for 5 min with the following acid fixing bath:

| | |
|---|---|
| water | 800 ml |
| anhydrous sodium thiosulphate | 200 g |
| (or sodium thiosulphate pentahydrate) | 300 g |
| potassium bisulphite | 12 g |
| glacial acetic acid | 12 ml |
| borax | 20 g |
| potassium alum | 15 g |
| water to make | 1 l |

The fixed elements were rinsed with water at 21°C for 10 min and then bleached for 7 min at 21°C in a bath having the following composition:

| | |
|---|---|
| potassium bromide | 20 g |
| potassium dichromate | 5 g |
| potassium alum | 40 g |
| water to make | 1 l |

The bleached elements were rinsed at 15°C for 5 min and fixed again for 5 min at 24°C in a same fixing bath. After having been rinsed again at 15°C for 10 min the elements were treated for 20 s in a stabilizing bath having the following composition:

| | |
|---|---|
| 5% saponin | 1.8 ml |
| 40% formaldehyde | 12.8 ml |
| water to make | 1 l |

Table 15 shows the results obtained for speed gradation, and maximum density of the exposed and processed elements comprising ballasted compounds C9, C10, C12, and M5 according to the invention as well as of the elements comprising analogous compounds (comparison) but carrying a known ballasting moiety (CR2 and MR3). The values given for speed are relative values, a value of 100 being given to the elements comprising the comparison compounds. The value 200 corresponds to a doubling of the speed.

TABLE 15

| Ballasted compound | Speed | Gradation | Maximum density |
|---|---|---|---|
| CR2 (comparison) | 100 | 1.7 | 2.8 |
| C9 | 102 | 2.6 | 3.4 |
| C10 | 100 | 2.9 | 3.4 |
| C12 | 100 | 2.4 | 3.0 |
| MR3 (comparison) | 100 | 1.4 | 3.2 |
| M5 | 123 | 1.9 | 3.2 |

The comparison couplers CR2 and MR3 correspond to the following structural formulae respectively:

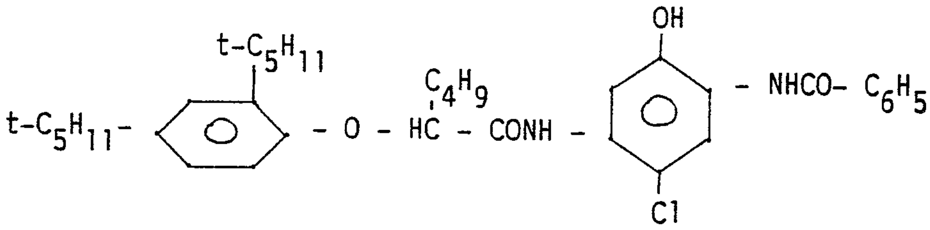

CR2

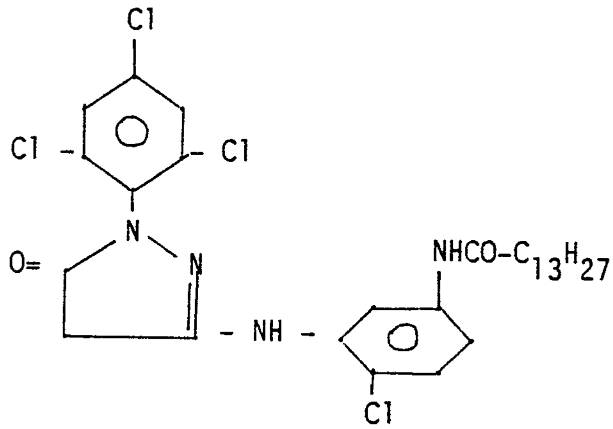

MR3

Comparison of the results listed in Table 15 shows that the gradation obtained with the ballasted couplers according to the invention is considerably higher than that of structurally analogous couplers - (comparison) comprising a conventional ballasting moiety.

The maximum density of the dye images obtained with the aid of couplers according to the invention is at least equal to and in most cases higher than that of the comparison couplers.

## EXAMPLE 3

Preparation of photosensitive element

A strip of subbed polyethylene terephthalate support having a thickness of 0.1 mm was coated with the following layers in the given order:

1) silver halide emulsion layer containing:

| | |
|---|---|
| gelatin | 1.8 g |
| silver chloride expressed as silver nitrate | 0.5 g |
| electron donor compound: | |
| 2,5-bis(1',1',3',3'-tetramethyl-butyl)-hydroquinone | 0.09 g |
| yellow dye-releasing compound D1 | |
| according to the following formula: | 0.333 g |

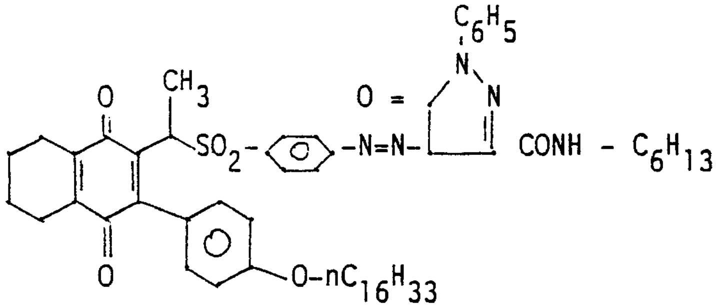

(D1)

2) protective layer containing:

| | | |
|---|---|---|
| gelatin | 6 | g |
| 1-phenyl-4-methyl-pyrazolidin-3-one | 0.12 | g |
| citric acid to adjust the pH to 4.5 in both layers | 0.06 | g |

Identical strips of photosensitive element were made, except that they contained instead of the above-mentioned dye-releasing compound D1 an equal molar concentration of the dye-releasing compound D2 corresponding to the following formula:

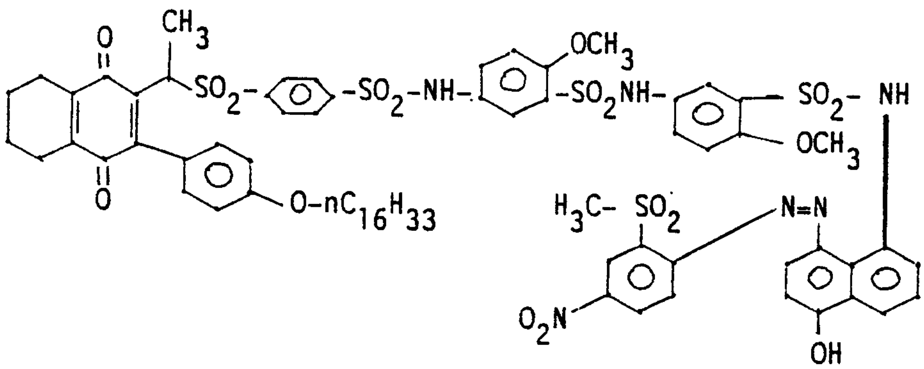

(D2)

Preparation of image-receiving element

Strips of corona-treated polyethylene-coated paper support were coated with the following image-receiving layer (first layer) and an antistress layer, the first layer of the strips comprising its ingredients in the concentrations indicated in Table 16:

1) first layer comprising gelatin, polymeric mordant prepared from 4,4'-diphenylmethane diisocyanate and N-ethyldiethanolamine quaternized with epichlorohydrin as described in Example 1 of DE-A 2,631,521, and the anti-fading agent A1,

2) antistress layer comprising gelatin.

An image-receiving element containing no anti-fading agent at all was used as comparison receptor. The image-receiving element containing the anti-fading agent A1 is called "test receptor" in Table 16.

The photosensitive elements were exposed image-wise and together with the image-receiving elements as described above fed through a COPYPROOF (trade mark of Agfa-Gevaert N.V. Belgium) CP 42 diffusion transfer processing apparatus containing in its tray an aqueous alkaline processing liquid comprising per litre:

| | |
|---|---|
| sodium hydroxide | 25 g |
| sodium orthophosphate | 25 g |
| cyclohexane dimethanol | 80 g |

| | |
|---|---|
| sodium bromide | 2 g |
| sodium thiosulphate | 2 g |
| water to make | 1 l |

After the diffusion transfer took place, the photosensitive elements were stripped from the image-receiving elements. The stability to light of the dyes transferred to the image-receiving elements and mordanted therein was tested with a XENOTEST (trade mark) type 50 apparatus of Hanau Quartzlampen GmbH, Hanau, W.Germany. The different strips of image-receiving element (receptor) containing the mordanted dyes were exposed for 8 h to white light and ultra-violet radiation. The % loss in maximum density of mordanted dye is given in Table 16.

TABLE 16

| Composition of receptor | | Amounts in g/m2 of | |
|---|---|---|---|
| | | Test receptor | Comparison receptor |
| 1st layer | gelatin | 3.6 | 4 |
| | polymeric mordant | 2.66 | 2.66 |
| | anti-fading compound A1 | 1 | - |
| antistress | gelatin | 0.66 | 0.66 |
| Mordanted dye deriving from dye-releasing compound | | % loss in maximum density | |
| D1 | | -13 | -27 |
| D2 | | -14 | -50 |

The results set forth in Table 16 show that when an anti-fading agent as defined above is made present together with dyes forming the colour images of diffusion transfer prints, the prevention from fading of these dyes is enhanced considerably.

## Claims

1. Photographic element, which in at least one hydrophilic colloid layer comprises at least one ballasted compound comprising a ballasting moiety that renders such compound substantially fast to diffusion in said hydrophilic colloid layer and at least one photographically useful moiety that is linked chemically to this ballasting moiety characterized in that the ballasting moiety of the ballasted compound corresponds to the following general formula I:

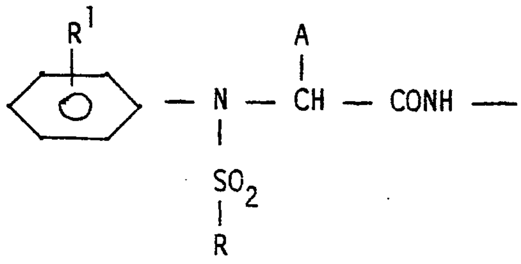

(I)

wherein:
A represents hydrogen, a straight chain or branched chain $C_1$-$C_{20}$ alkyl group, or an aryl group;
R is a $C_1$-$C_{20}$ alkyl group;
$R^1$ represents hydrogen, hydroxy, sulpho, carboxy, cyano, a halogen atom, trifluoromethyl, a hydroxyalkyl group, a sulphoalkyl group, a carboxyalkyl group, an alkyl group, an alkoxy group, an alkylthio group, an acyl group, $R^2CONH$-, $R^2NHCO$-, $R^2NHSO_2$ , or $R^2SO_2N(R^3)$-, each of $R^2$ and $R^3$ being hydrogen, a $C_1$-$C_5$ alkyl group, or an aryl group and when both present having a same or a different significance.

2. A photographic element according to claim 1, characterized in that the photographically useful moiety is a coupler.

3. A photographic element according to claim 1, characterized in that the photographically useful moiety is an anti-fading moiety.

4. Compounds comprising a ballasting moiety that renders such compounds substantially fast to diffusion in a hydrophilic colloid medium characterized in that the ballasting moiety corresponds to the general formula I defined in claim 1.

European Patent Office

# EUROPEAN SEARCH REPORT

Application number

EP 85 20 1961

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| Y | GB-A-2 066 811 (KONISHIROKU PHOTO)<br>* Page 1, line 42 - page 3, line 7; claims *<br>--- | 1,2,4 | G 03 C 7/32<br>G 03 C 5/54<br>C 07 D 231/52<br>C 07 D 211/58 |
| Y | FR-A-2 412 525 (FISONS)<br>* Page 24, line 8 - page 25, line 29; page 39, example 40 *<br>--- | 1 | |
| A | GB-A-2 066 494 (FUJI PHOTO FILM)<br>----- | | |
| | | | TECHNICAL FIELDS SEARCHED (Int. Cl.4) |
| | | | G 03 C 7 |

The present search report has been drawn up for all claims

| Place of search | Date of completion of the search | Examiner |
| --- | --- | --- |
| THE HAGUE | 08-07-1986 | PHILOSOPH L.P. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document